# EUROPEAN PATENT APPLICATION

(11) **EP 2 286 787 A2**
(43) Date of publication of application: **23.02.2011**
(21) Application number: 10172936.6
(22) Date of filing: 16.08.2010
(51) Int. Cl.: A61K 8/34, A61K 8/36, A61K 8/86, A61Q 9/00, B26B 21/44

(54) **Hair removal device**

(30) Priority: 21.08.2009 EP 09168356
(71) Applicant: The Gillette Company, Boston, MA 02127 (US)
(72) Inventor: Stephens, Alison, Fiona, Maidenhead, Berkshire SL6 9LA (GB); Geary, Elaine, Alice, Marie, St Margarets, Middlesex TW1 2QX (GB); Havas, Fabien, Isleworth, Middlesex TW7 4JG (GB)
(74) Representative: Wilding, Richard Alan

(57) **Abstract**

A hair removal device is provided comprising a cold-pressed, solid lubricating composition, the cold-pressed, solid lubricating composition comprising a temperature-sensitive component, such as fragrance oil.

## Description

### FIELD OF THE INVENTION

The present invention concerns the provision of a hair removal device comprising solid cosmetic compositions comprising heat sensitive components.

### BACKGROUND OF THE INVENTION

It is known to provide a hair removal device incorporating a skin-engaging solid cosmetic composition comprising hydrophilic polymers to lubricate the skin. Typically, such solid compositions are manufactured in a high temperature process involving heating to a temperature sufficient to melt all the components, then co-mixing in an extruder. Reference is made, by way of example, to WO 97/02116 and WO 97/02117. Such a process has a number of disadvantages. In the first place, it necessitates actively heating to above the melting point of the highest melting point material, which is often above about 180°C. This is very energy intensive and therefore costly and environmentally unfriendly. Furthermore, temperature-sensitive materials, such as some perfume oils, may flash off and be lost to the formulation. Fragrance compositions usually comprise a combination of different perfume oils which, together, provide a balanced impression to the user, but that balance may be lost if even just one or two of the perfume oils are lost by boiling off during manufacture. Other materials, such as menthol, a sensate, which sublimes at low temperatures, may similarly be lost. Yet other materials may become degraded, modified or destroyed. All of these effects may result in a formulation which has a different composition from the intended one. These effects are also wasteful and costly.

### SUMMARY OF THE INVENTION

According to a first aspect of the invention a hair removal device is provided comprising a cold-pressed, solid lubricating composition, the cold-pressed, solid lubricating composition comprising a temperature-sensitive component, such as fragrance oil.

According to a second aspect of the invention a process for manufacturing a hair removal device comprising a solid lubricating composition is provided, the process comprising the steps of:
a. providing a water-soluble lubricating polymer in finely divided form which;
   i. is solid at STP;
   ii. has a molecular weight of less than or equal to 8,000,000, preferably from 100,000 to 3,000,000.
b. providing a temperature-sensitive component, such as fragrance oil;
c. intimately mixing the water-soluble lubricating polymer and the temperature-sensitive component;
d. compressing the mixture without active heating to provide a solid, lubricating composition;
e. providing a hair removal device;
f. affixing the solid lubricating composition to the hair removal device.

### DETAILED DESCRIPTION OF THE INVENTION

The present invention overcomes problems associated with prior art hair removal devices which comprise compositions manufactured at high temperatures. The solution involves cold-pressing to manufacture the composition according to the first aspect of the invention.

The hair removal device according to the invention may be any hair removal device, such as, but not limited to, a razor or a hair removal device comprising a light source to degrade or destroy the hair. If the hair removal device comprises a light source, then the light source is advantageously a source of laser light. Preferably, the hair removal device is a razor. In the case of a razor, then the solid lubricating composition may advantageously be provided on the razor cartridge, and is preferably as a strip located before and/or after the blade(s) in the direction of cutting. WO 97/02116, referred to above, illustrates locations in which such a strip may be placed.

As used herein, the term "STP" or "Standard Temperature and Pressure" refers to a temperature of 20 °C and a pressure of 101.325 kPa.

As used herein, the term "cold-pressed" means that the formulation has been manufactured by process involving a compression step, without any active heating and the term "cold-pressing" should be interpreted accordingly. For the avoidance of doubt, heat generated by the compression step itself is not regarded as being active heating.

The term "temperature-sensitive component" refers to materials which have a boiling point, undergo significant sublimation, or which undergo a change in chemical structure at temperatures above ambient temperatures and below 100°C. Materials having a boiling point in this range are known to the skilled person and include many low boiling point perfume oils. Low boiling perfume oils, or top notes, are those that provide the initial burst of fragrance following application to the skin, because they boil off quickly when exposed to body heat. The skilled person is similarly familiar with materials which undergo significant sublimation in the present temperature range. An example of a commonly used sensate which sublimes in this temperatures range is menthol. Materials whose chemical structures change at temperatures within the cited range are known to the skilled person. One example of such a material is L-ascorbic acid, which oxidizes at an increasing rate as the temperature rises.

The cold-pressed, solid lubricating composition, may comprise a water-soluble lubricating polymer. The water-soluble lubricating polymer may achieve lubrication by a number of mechanisms, such as binding water to form a gel. In order to be effective, the polymer must be solid at STP and have a molecular weight of less than 8,000,000. Preferably, it has a molecular weight from 100,000 to 7,000,000. Suitable water-soluble lubricating polymers include polyalkylene glycol; cellulosic polymers, including hydroxypropyl cellulose; hydroxypropyl methylcellulose (HPMC); polyvinyl pyrrolidone; polyacrylamide; polyvinyl imidazoline; polyhydroxyethylmethacrylate and mixtures thereof. As used herein, "polyalkylene glycol" is synonymous with "polyalkane oxide", although some sources have, in the past, referred to lower molecular weight variants as "polyalkylene glycol" and higher molecular weight variants as "polyalkane oxide". Preferably, the polyalkylene glycol (polyalkane oxide) comprises polyethylene glycol (which may also be referred to herein as "PEG"), polypropylene glycol, or mixtures thereof and more preferably it comprises polyethylene glycol.

The cold-pressed, solid lubricating composition preferably comprises from 20 to 80%, more preferably about 40 to 75%, by weight of water-soluble lubricating polymer.

Advantageously, the water-soluble lubricating polymers comprise polyethylene oxides generally known as POLYOX (available from The Dow Chemical Company) or ALKOX (available from Meisei Chemical Works, Kyoto, Japan). In one embodiment, these particular polyethylene oxides will have molecular weights of about 100,000 to about 6 million, preferably from about 300,000 to about 5 million and the polyethylene oxide comprises a blend of polyethylene oxide having an average molecular weight of about 5 million and polyethylene oxide having an average molecular weight of about 300,000.

Advantageously, the solid lubricating composition comprises less than 2%, preferably less than 1% and more preferably 0% of water-soluble surfactant, by weight of the solid lubricating composition. As used herein, the term "water-soluble surfactant" refers to surfactant which fulfils the following condition: at 25°C, 1g of the surfactant dissolves in 1000ml or less of water. For completeness, one requires more than 10,000ml of water at 25°C in order to dissolve 1g of magnesium stearate or 1g of cetyl alcohol.

Advantageously, cold-pressed, solid lubricating compositions may comprise a hydrophobic binder. The presence of such a component may enhance the life of the composition by reducing its tendency to be mechanically eroded. Advantageously, the hydrophobic binder is solid at STP. Suitable hydrophobic binders include divalent metal cation stearate, preferably magnesium stearatte, calcium stearate, zinc stearate, or mixtures thereof, more preferably magnesium stearate; ethyl cellulose, polycaprolactone and mixtures thereof. Preferably, the cold-pressed, solid lubricating composition comprises from 1 to 20% and more preferably from 5 to 15% hydrophobic binder, by weight of the cold-pressed, solid lubricating composition.

Advantageously, cold-pressed, solid lubricating compositions may comprise a C₁₂ - C₂₂ fatty alcohol, or mixtures thereof, preferably cetyl, stearyl, behenyl alcohol or mixtures thereof. The presence of a fatty alcohol may enhance the life of the composition by reducing its tendency to be dissolved in water. Preferably, the cold-pressed, solid lubricating composition comprises from 1 to 20% and more preferably from 5 to 15% fatty alcohol, by weight of the cold-pressed, solid lubricating composition.

It may be difficult to provide a perfumed a solid product of the present type, because perfumes are liquids and are therefore difficult to distribute evenly throughout the solid matrix during a cold-pressing process. Furthermore, perfume oils are usually fairly hydrophobic and may be repelled by hydrophilic materials, such as the water-soluble lubricating polymers disclosed herein. This fact may also contribute to perfume oils becoming squeezed out of the solid composition during compaction, thereby reducing the degree of perfuming and wasting raw material. The result tends to be an unsatisfactorily perfumed product.

In order to overcome this disadvantage, perfume oils comprised within the cold-pressed, solid lubricating compositions according to the invention may be releasably encapsulated within a powder. Any powder which permits perfume release in use, such as on contact with water, may suitably be used. Advantageously, the powder comprises a cyclic oligosaccharide, which may encapsulate the perfume.

As used herein, the term "cyclic oligosaccharide" means a cyclic structure comprising six or more saccharide units. Preferred for use herein are cyclic oligosaccharides having six, seven or eight saccharide units and mixtures thereof, more preferably six or seven saccharide units and even more preferably seven saccharide units. It is common in the art to abbreviate six, seven and eight membered cyclic oligosaccharides to α, β and γ respectively.

Cyclic oligosaccharides for use herein may comprise any suitable saccharide or mixtures of saccharides. Examples of suitable saccharides include, but are not limited to, glucose, fructose, mannose, galactose, maltose and mixtures thereof, preferably glucose. The preferred cyclic oligosaccharides for use herein are α-cyclodextrins or β- cyclodextrins, or mixtures thereof, and the most preferred cyclic oligosaccharides for use herein are β-cyclodextrins.

The cyclic oligosaccharides for use herein may be substituted by any suitable substituent or mixture of substituents. Herein the use of the term "mixture of substituents" means that two or more different suitable substituents can be substituted onto one cyclic oligosaccharide. The derivatives of cyclodextrins consist mainly of molecules wherein some of the OH groups have been substituted. Suitable substituents include, but are not limited to, alkyl groups; hydroxyalkyl groups; dihydroxyalkyl groups; (hydroxyalkyl) alkylenyl bridging groups such as cyclodextrin glycerol ethers; aryl groups; maltosyl groups; allyl groups; benzyl groups; alkanoyl groups; cationic cyclodextrins such as those containing 2-hydroxy-3- (dimethylamino) propyl ether; quaternary ammonium groups; anionic cyclodextrins such as carboxyalkyl groups, sulphobutylether groups, sulphate groups, and succinylates; amphoteric cyclodextrins; and mixtures thereof.

The substituents may be saturated or unsaturated, straight or branched chain. Preferred substituents include saturated and straight chain alkyl groups, hydroxyalkyl groups and mixtures thereof. Preferred alkyl and hydroxyalkyl substituents are selected from Cl-C8 alkyl or hydroxyalkyl groups or mixtures thereof, more preferred alkyl and hydroxyalkyl substituents are selected from Cl-C6 alkyl or hydroxyalkyl groups or mixtures thereof, even more preferred alkyl and hydroxyalkyl substituents are selected from Cl-C4 alkyl or hydroxyalkyl groups and mixtures thereof. Especially preferred alkyl and hydroxyalkyl substituents are propyl, ethyl and methyl, more especially hydroxypropyl and methyl and even more preferably methyl.

Preferred cyclic oligosaccharides for use in the present invention are unsubstituted, or are substituted by only saturated straight chain alkyl, or hydroxyalkyl substituents. Therefore, preferred examples of cyclic oligosaccharides for use herein are α-cyclodextrin, β-cyclodextrin, methyl-α-cyclodextrin, methyl-α-cyclodextrin, and hydroxypropyl-β-cyclodextrin.

The following, non-limiting examples, illustrate the manufacture of cold-pressed, solid lubricating compositions:

### Example 1

| **Component** | **Wt%** |
|---|---|
| | |
| PEG¹ | 48 |
| Sodium carboxymethyl cellulose | 25 |
| Magnesium stearate | 2 |
| Cetyl alcohol² | 15 |
| Menthol | 10 |

| | |
|---|---|
| ¹Polyox^{™} WSR-N750 powder, a polyethylene glycol with molecular weigh about 300,000 manufactured by Dow Chemicals ²Lanette 16 powder, manufactured by Cognis | |

### Example 2

| **Component** | **Wt%** |
|---|---|
| | |
| PEG¹ | 80 |
| Hydroxypropylmethyl cellulose | 5 |
| Cetyl alcohol² | 5 |
| Beta-cyclodextrin/fragrance encapsulate | 10 |

The above composition is manufactured by placing all materials into a blending device and blending until the mixture is entirely homogeneous. Once homogenised, the mixture is pressed into the desired shape using an appropriate mould tool / punch. An example of a suitable device is the GEA Courtoy R253-27 tablet press. The powder is compressed using a force of 1 x 10¹⁰ - 1 x 10¹² Nm⁻². The resultant tablet is ejected from the mould in the desired shape.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40mm" is intended to mean "about 40mm".

## Claims

1. A hair removal device comprising a cold-pressed, solid lubricating composition, the cold-pressed, solid lubricating composition comprising a temperature-sensitive component, such as fragrance oil.

2. The hair removal device of claim 1, wherein the cold-pressed, solid lubricating composition comprises a water-soluble lubricating polymer which:
i. is solid at STP;
ii. has a molecular weight of less than or equal to 8,000,000, preferably from 100,000 to 7,000,000.

3. The hair removal device of claim 2, wherein the water-soluble lubricating polymer comprises polyoxyalkylene glycol, polyalkane oxide, cellulosic polymer, polyvinyl pyrrolidone, polyacrylamide, polyvinyl imidazoline, polyhydroxyethylmethacrylate or mixtures thereof.

4. The hair removal device of any preceding claim, wherein the cold-pressed, solid lubricating composition additionally comprising a hydrophobic binder.

5. The hair removal device of claim 4, wherein the hydrophobic binder comprises magnesium stearate, calcium stearate, zinc stearate, ethyl cellulose, polycaprolactone or mixtures thereof.

6. The hair removal device of any preceding claim, wherein the cold-pressed, solid lubricating composition additionally comprises a C₁₂ - C₂₂ fatty alcohol, or mixtures thereof.

7. The hair removal device of any preceding claim, wherein the cold-pressed, solid lubricating composition comprises fragrance oil releasably, encapsulated within a cyclic oligosaccharide.

8. The hair removal device of claim 7, wherein the cyclic oligosaccharide comprises α-cyclodextrin, β-cyclodextrin, methyl-α-cyclodextrin, methyl-α-cyclodextrin, hydroxypropyl-β-cyclodextrin, or mixtures thereof.

9. The hair removal device of any preceding claim, configured as a razor.

10. The hair removal device of any of claims 1 to 8, wherein the hair removal device comprises a light source to degrade or destroy the hair or hair root.

11. The hair removal device of claim 10, wherein the light source comprises a laser light source.

12. Use of the hair removal device of any preceding claim to lubricate the skin and remove hair.

13. A process for manufacturing a hair removal device comprising a solid lubricating composition, the process comprising the steps of:
a. providing a water-soluble lubricating polymer in finely divided form which;
i. is solid at STP;
ii. has a molecular weight of less than or equal to 8,000,000, preferably from 100,000 to 3,000,000.
b. providing a temperature-sensitive component, such as fragrance oil;
c. intimately mixing the water-soluble lubricating polymer and the temperature-sensitive component;
d. compressing the mixture without active heating to provide a solid, lubricating composition;
e. providing a hair removal device;
f. affixing the solid lubricating composition to the hair removal device.
